(19) **Europäisches Patentamt / European Patent Office / Office européen des brevets**

(11) **EP 4 412 801 B1**

(12) **FASCICULE DE BREVET EUROPEEN**

(45) Date de publication et mention de la délivrance du brevet:
**03.09.2025 Bulletin 2025/36**

(21) Numéro de dépôt: **22814479.6**

(22) Date de dépôt: **06.10.2022**

(51) Classification Internationale des Brevets (IPC):
**B25J 9/00** *(2006.01)*     **A61H 3/00** *(2006.01)*

(52) Classification Coopérative des Brevets (CPC):
**B25J 9/0006;** A61H 1/0237; A61H 3/00;
A61H 2003/007

(86) Numéro de dépôt international:
**PCT/FR2022/051894**

(87) Numéro de publication internationale:
**WO 2023/057727 (13.04.2023 Gazette 2023/15)**

(54) **PROCEDE DE MISE EN MOUVEMENT D'UN EXOSQUELETTE**

VERFAHREN ZUM BEWEGEN EINES EXOSKELETTS

METHOD FOR MOVING AN EXOSKELETON

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **06.10.2021 FR 2110555**

(43) Date de publication de la demande:
**14.08.2024 Bulletin 2024/33**

(73) Titulaire: **Wandercraft**
**75004 Paris (FR)**

(72) Inventeurs:
• **HUYNH, Vaiyee**
  **94110 ARCUEIL (FR)**
• **EL KHOURY, Antonio**
  **94114 San Francisco (US)**

(74) Mandataire: **Regimbeau**
**20, rue de Chazelles**
**75847 Paris Cedex 17 (FR)**

(56) Documents cités:
**WO-A2-2015/140353**

• **PENCO LUIGI ET AL: "Learning Robust Task Priorities and Gains for Control of Redundant Robots", IEEE ROBOTICS AND AUTOMATION LETTERS, IEEE, vol. 5, no. 2, 10 February 2020 (2020-02-10), pages 2626 - 2633, XP011774741, DOI: 10.1109/LRA.2020.2972847**

## Description

DOMAINE TECHNIQUE GENERAL

La présente invention concerne le domaine des robots de type exosquelette.

Plus précisément, elle concerne un procédé de mise en mouvement d'un exosquelette, dans un mode dit d'exercice.

ETAT DE L'ART

Récemment, sont apparus pour les personnes avec des problèmes de mobilité importants comme les paraplégiques des dispositifs de marche assistée appelés exosquelettes, qui sont des dispositifs robotisés externes que l'opérateur (l'utilisateur humain) vient « enfiler » grâce à un système d'attaches qui lie les mouvements de l'exosquelette de ses propres mouvements. Les exosquelettes de membres inférieurs disposent de plusieurs articulations, généralement au moins au niveau des genoux et des hanches, pour reproduire le mouvement de marche. Des actionneurs permettent de mouvoir ces articulations, qui à leur tour font se mouvoir l'opérateur. Un système d'interface permet à l'opérateur de donner des ordres à l'exosquelette, et un système de commande transforme ces ordres en commande pour les actionneurs. Des capteurs viennent généralement compléter le dispositif.

Ces exosquelettes constituent une avancée par rapport aux fauteuils roulants, car ils permettent aux opérateurs de se remettre debout et de marcher. Les exosquelettes ne sont plus limités par les roues et peuvent théoriquement évoluer dans la majorité des environnements urbains : les roues, au contraire des jambes, ne permettent pas de franchir des obstacles importants comme des marches, escaliers, obstacles d'une hauteur trop importante, etc.

Outre la récupération de mobilité, les exosquelettes ont un fort intérêt en rééducation, en particulier suite à des accidents neurologiques tels que des AVC.

La « verticalisation » est en particulier un exercice important dans la rééducation des personnes à mobilité réduite. Elle un impact significatif important sur la personne, que ce soit psychologique ou physique. Cet exercice leur permet d'une part, se mettre à hauteur des personnes de leur entourage pour interagir socialement, ce qui est très bon pour leur moral (confiance en soi et dignité). D'autre part, elle permet d'améliorer la respiration, la circulation sanguine ou encore la digestion, et d'éviter la rétraction musculaire et l'apparition d'escarres.

On appelle mode « exercice » un mode de fonctionnement d'un exosquelette dans lequel le patient ne se déplace pas, et ses deux pieds restent toujours en contact avec le sol (ils sont immobiles), afin de réaliser des mouvements d'exercice d'intérêt pour la rééducation du patient et/ou pour la dynamisation du corps.

Le problème est qu'un paraplégique ne peut pas faire de mouvement qui sera « suivi » par l'exosquelette. Dans le cas d'une marche, l'exosquelette peut appliquer une trajectoire prédéfinie, mais cela n'est plus possible dans le mode exercice, car alors le mouvement doit pouvoir être complètement contrôlé par le patient, à partir de données capteur.

On connait un seul exosquelette qui permet à un paraplégique de réaliser des exercices en posture debout, il s'agit du REX de RexBionics. Cependant, il fonctionne selon un principe simpliste : l'exosquelette se fige dans une posture donnée, et ensuite le patient peut mobiliser le haut du corps, mais sans aucune possibilité de contrôle l'exosquelette.

On comprend que cette solution limite fortement les mouvements d'exercice possibles : le mode « Rexercices » de REX permet uniquement de manipuler des ballons et des équipements de musculation de la partie haute du corps, alors qu'il serait profitable de permettre au patient d'effectuer des squats, de se pencher pour attraper un objet, de bouger les hanches, etc.

De surcroit, maintenir le patient dans une posture statique pose quelques problèmes :

- Sensation d'être bloqué dans l'exosquelette ;
- Risque de basculement du robot avec les mouvements amples du patient et par conséquent, le blesser ;
- Non-atteinte des objets situés à basse altitude.

Au contraire, dynamiser la verticalisation et augmenter l'espace d'atteinte permet de motiver le patient dans cet exercice et de lui donner le contrôle de ses mouvements.

Il serait souhaitable de disposer d'une nouvelle solution de mise en mouvement d'un exosquelette, qui permette une plus grande variété d'exercices tout en restant stable et sécurisé pour le patient.

Par ailleurs, on connaît notamment de L. Penco, E. M. Hoffman, V. Modugno, W. Gomes, J. -B. Mouret and S. Ivaldi, "Learning Robust Task Priorities and Gains for Control of Redundant Robots," in IEEE Robotics and Automation Letters, vol. 5, no. 2, pp. 2626-2633, April 2020, des procédés de commande de robots bipèdes mettant en œuvre des piles de tâches hiérarchisées.

PRESENTATION DE L'INVENTION

La présente invention se rapporte ainsi selon un premier aspect à un procédé de mise en mouvement d'un exosquelette bipède recevant un opérateur humain, le procédé étant caractérisé en ce qu'il comprend la mise en œuvre par des moyens de traitement de données de l'exosquelette, d'étapes de :

(a) obtention d'au moins une consigne posturale à appliquer par l'exosquelette pour que l'opérateur réalise un mouvement d'exercice ;
(b) détermination d'une trajectoire de l'exosquelette par la mise en œuvre d'une cinématique inverse hiérarchisée en fonction de l'au moins une consigne posturale déterminée, ladite cinématique inverse hiérarchisée présentant une pile de tâches hiérarchisées comprenant, comme tâche de plus forte priorité, une tâche de maintien des pieds de l'exosquelette au sol lors dudit mouvement d'exercice de l'opérateur.

Selon des caractéristiques avantageuses et non-limitatives :

La pile de tâches hiérarchisées comprend en outre au moins une tâche de contrôle du reste de l'exosquelette, de priorité moindre que ladite tâche de maintien des pieds de l'exosquelette au sol.

Ladite au moins une tâche de contrôle du reste de l'exosquelette est choisie parmi :

- une tâche de contrôle du centre de masse, CoM, de l'exosquelette ;
- une tâche de contrôle du bassin de l'exosquelette ;
- une tâche de contrôle de la posture de l'exosquelette.

La pile de tâches hiérarchisées comprend, par ordre de priorité décroissant, ladite tâche de maintien des pieds de l'exosquelette au sol, la tâche de contrôle du CoM de l'exosquelette, la tâche de contrôle du bassin de l'exosquelette, et la tâche de contrôle de la posture de l'exosquelette.

La tâche de contrôle du CoM de l'exosquelette est une tâche de contrôle en position et de stabilisation du CoM via un contrôleur basé sur un modèle de pendule inversé flexible.

L'étape (b) comprend l'exécution de boucles de contrôle définissant pour chaque tâche l'évolution d'une position de l'exosquelette de sorte à mettre en œuvre ladite trajectoire déterminée.

On a une boucle de contrôle indépendante par tâche, la cinématique inverse hiérarchisée étant mise en œuvre au sein desdites boucles de contrôle.

La position de l'exosquelette est définie par un vecteur des positions articulaires des degrés de liberté actionnés de l'exosquelette.

L'étape (a) comprend l'identification d'une intention de mouvement par l'opérateur, à partir de données acquises par des capteurs de l'exosquelette ; et la détermination de l'au moins une consigne posturale à partir de ladite intention de mouvement.

L'au moins une consigne posturale est une consigne de centre de masse, CoM, et/ou d'articulation définissant une pose désirée de l'exosquelette lors dudit mouvement d'exercice de l'opérateur.

Selon un deuxième aspect, l'invention concerne un exosquelette comprenant des moyens de traitement de données configurés pour mettre en œuvre un procédé selon le premier aspect de mise en mouvement de l'exosquelette.

Selon un troisième et un quatrième aspect, l'invention concerne un produit programme d'ordinateur comprenant des instructions de code pour l'exécution d'un procédé selon le premier aspect de mise en mouvement d'un exosquelette ; et un moyen de stockage lisible par un équipement informatique sur lequel un produit programme d'ordinateur comprend des instructions de code pour l'exécution d'un procédé selon le premier aspect de mise en mouvement d'un exosquelette.

## PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :

- **la figure 1** est un schéma d'un exosquelette utilisé par les procédés selon l'invention ;
- **la figure 2** est un diagramme illustrant un mode de réalisation préféré du procédé selon l'invention ;
- **la figure 3a** représente schématiquement une première boucle de contrôle utilisée dans un mode de réalisation préféré du procédé selon l'invention ;
- **la figure 3b** représente schématiquement une deuxième boucle de contrôle utilisée dans un mode de réalisation préféré du procédé selon l'invention.

## DESCRIPTION DETAILLEE

Architecture

La présente invention propose un procédé de mise en mouvement d'un exosquelette 1.

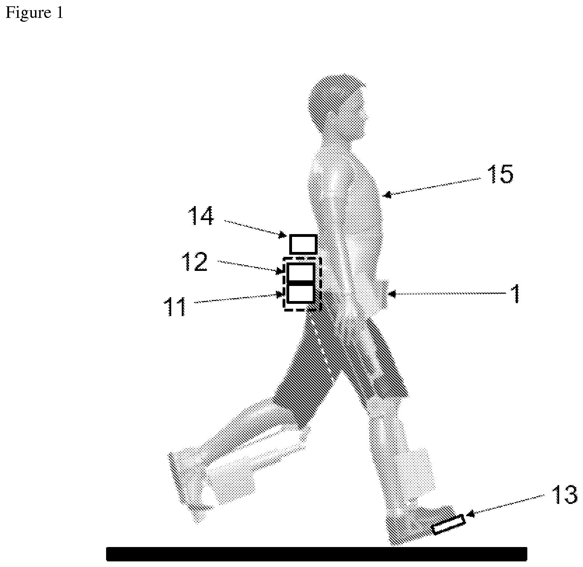

En référence à la **figure 1,** ledit exosquelette 1 est un système mécanique articulé de type dispositif robotisé bipède, actionné et commandé, pourvu de deux jambes, accueillant plus précisément un opérateur humain présentant ses membres inférieurs chacun solidaires d'une jambe de l'exosquelette 1 (notamment grâce à des sangles). Il peut ainsi être un robot plus ou moins humanoïde.

On fera ici une différence entre le mouvement de l'opérateur (qui est un mouvement global qui implique le plus souvent des mouvements des bras et de la partie haute du corps que l'exosquelette subit, et qu'on appellera « mouvement d'exercice » dans le cadre de la présente invention) et le mouvement de l'exosquelette 1 seul ou « trajectoire » (qui est donc limité aux jambes). En pratique c'est l'opérateur qui réalise l'exercice et donc bouge physiquement le haut du corps, et l'exosquelette 1 répond en mettant en œuvre une trajectoire lors de laquelle les pieds de l'exosquelette 1 restent immobiles, en contact avec le sol.

En d'autres termes, dans le présent mode exercice, l'exosquelette 1 est mis en mouvement en maintenant les pieds de l'exosquelette 1 fixes. On comprend qu'il s'agit d'une contrainte imposée par le mode exercice (i.e. le maintien des pieds au sol est imposé par l'exosquelette 1 lorsqu'il est dans ledit mode exercice).

On comprend que le mouvement « lors duquel les pieds de l'exosquelette 1 restent en contact du sol » s'entend par opposition à un mouvement de type « marche » qui se traduit en pratique par un appui alternatif sur les jambes, en position debout, de sorte à produire un déplacement. Typiquement un mouvement de marche de l'exosquelette est composé d'une séquence de pas, chaque pas voyant un pied se décoller du sol puis se reposer, avant inversion des rôles (i.e. une alternance de pas du pied gauche et du pied droit).

Ainsi, on n'a dans le cadre du présent procédé aucun déplacement de l'exosquelette 1 du fait de l'immobilité des pieds, mais l'exosquelette 1 reste en mouvement (et tous ses degrés de libertés sont mobiles), contrairement à ce qui était le cas par exemple pour REX, et vient accompagner l'opérateur. Le mode exercice a comme objectif général d'augmenter l'espace d'atteinte du patient tout en le stabilisant debout lorsqu'il bouge le haut de son corps. On comprend en effet, qu'il est par exemple indispensable que les genoux de l'exosquelette 1 se plient pour que l'utilisateur puisse ramasser quelque chose posé par terre devant lui.

Ce mouvement d'exercice réalisé par l'opérateur pourra par exemple être :

- soulever/tirer/attraper/lancer un objet avec un ou deux bras ;
- se pencher, éventuellement pour ramasser quelque chose ;
- faire des mouvements de rotation du bassin sur un ou plusieurs axes, le mouvement de « roulis » du bassin (rotation d'axe longitudinal) étant communément appelé « déhanché » ;
- faire des squats ;
- faire des étirements ;
- pratiquer un sport (sport de raquette, boxe, basketball, etc.) ;
- etc.

Cependant, on comprendra que tout mouvement d'exercice lors duquel les pieds de l'exosquelette 1 restent fixes pourra être effectué sans limitation dans le cadre de la présente invention, tant qu'il existe une façon d'accomplir ce mouvement de manière stable.

L'exosquelette 1 présente une pluralité de degrés de liberté, c'est-à-dire d'articulations déformables (généralement via une rotation) c'est-à-dire mobiles les unes par rapport aux autres qui sont chacun soit « actionné », soit « non-actionné ». Un degré de liberté actionné désigne une articulation pourvue d'un actionneur commandé par des moyens de traitement de données 11, c'est-à-dire que ce degré de liberté est contrôlé et que l'on peut agir dessus. Comme l'on verra, certains de ces degrés de liberté peuvent être « flexibles ».

Les moyens de traitement de données 11 désignent un équipement informatique (typiquement un processeur, soit externe si l'exosquelette 1 est « télécommandé » mais préférentiellement embarqué dans l'exosquelette 1, voir plus loin) adapté pour traiter des instructions et générer des commandes à destination des différents actionneurs. Ces derniers peuvent être électriques, hydrauliques, etc.

L'exosquelette 1 peut en outre comprendre des moyens de stockage de données 12, des moyens de mesure inertielle 14 (centrale à inertie), des moyens pour détecter l'impact des pieds au sol 13 et le cas échéant estimer les forces de contact (capteurs de contact ou éventuellement capteurs de pression), et/ou un gilet muni de capteurs 15.

La présente demande ne sera limitée à aucune architecture d'exosquelette 1, et on prendra l'exemple tel que décrit dans les demandes WO2015140352 et WO2015140353.

Ainsi, de façon préférée et conformément à ces demandes, l'exosquelette 1 comprend sur chaque jambe une structure de pied comprenant un plan de support sur lequel un pied d'une jambe de la personne portant l'exosquelette peut venir en appui.

Lorsque qu'on parle de « pied de l'exosquelette 1 restant en contact du sol », on comprend qu'on désigne cette structure de pied.

L'homme du métier saura toutefois adapter le présent procédé à toute autre architecture mécanique, et il suffit que l'exosquelette dispose de deux jambes chacune terminée d'un pied.

Principe

On entend classiquement par « trajectoire » de l'exosquelette les évolutions de chaque degré de liberté (en particulier actionné, mais les degrés non actionnés peuvent intervenir dans les algorithmes de commande des autres degrés de liberté) exprimées en fonction du temps ou d'une variable de phase. Dans la suite de la présente description, par « position » de l'exosquelette 1 on entendra les positions articulaires des degrés de liberté actionnés, qui sont avantageusement au nombre de six par jambe, soit une position définie par un vecteur de dimension 12.

On comprend que la trajectoire présente ici la contrainte d'avoir les pieds fixes, mais tous les degrés de libertés continuent d'avoir une évolution.

Pour respecter cette contrainte, l'invention propose de déterminer la trajectoire par la mise en œuvre d'une cinématique inverse « hiérarchisée », i.e. présentant une pluralité de tâches hiérarchisées.

La cinématique inverse (souvent abrégée IK, de l'anglais « inverse kinematics ») désigne une solution de calcul de la « position » de l'exosquelette 1 (i.e. une configuration de toutes ses positions articulaires comme expliqué) afin d'obtenir une pose désirée. Le terme cinématique inverse renvoie au fait que la résolution des calculs est généralement basée sur les équations cinématiques du modèle articulaire.

On appelle « tâche » un objectif de la cinématique inverse définissant tout ou partie de la pose désirée (on peut ainsi définir la pose désirée comme une pluralité de tâches découplées), et on sait obtenir une loi de contrôle en « empilant » un certain nombre de tâches hiérarchisées de la plus prioritaire à la moins prioritaire. On parle dans la littérature de SoT pour « Stack of Tasks ».

La résolution de la cinématique inverse est généralement complexe d'un point de vue calculatoire, a fortiori si elle comprend une pluralité de tâches hiérarchisées, même si l'on connait aujourd'hui des algorithmes de cinématique inverse hiérarchisée performants, voir par exemple le document A Dedicated Quadradic Program for Fast Hierarchical-Inverse-Kinematic Resolution. A. Escande, N. Mansard and P-B. Wieber. In IEEE Int. Conf. on Robotics and Automation (ICRA'10), Anchorage, USA, May 2010.

La présente invention utilise de manière très astucieuse la cinématique inverse hiérarchisée pour le mode exercice en prenant comme tâche de plus forte priorité (tâche 0) une tâche de maintien fixe des pieds de l'exosquelette 1. Plus précisément, ladite tâche de plus forte priorité assure le contrôle des pieds de l'exosquelette 1, avantageusement en position et rotation (6D), et a pour consigne que ces pieds ne doivent pas bouger. Dans la mesure où c'est la tâche la plus prioritaire, il s'agit de la contrainte la plus forte, et on garantit ainsi que les pieds de l'exosquelette 1 restent au sol, quitte à ce que des tâches moins prioritaires ne soient pas pleinement accomplies, i.e. que l'exosquelette n'ait pas exactement la posture escomptée. Ainsi, on peut se permettre de ne pas bloquer tout l'exosquelette 1 comme dans l'art antérieur.

Naturellement, la cinématique inverse hiérarchisée comprend en outre au moins une autre tâche de contrôle du reste de l'exosquelette 1, de priorité moindre, avantageusement deux, voire trois, préférentiellement choisie(s) parmi :

- une tâche de contrôle (en position) du centre de masse (CoM) de l'exosquelette 1, en particulier pour stabilisation ;
- une tâche de contrôle (en rotation) du bassin de l'exosquelette 1, pour rendre le mouvement plus anthropomorphe ;
- une tâche de contrôle de la posture de l'exosquelette 1, pour permettre à l'algorithme de converger plus rapidement vers une solution viable.

De manière préférée, la cinématique inverse hiérarchisée comprend ces quatre tâches hiérarchisées par ordre de priorité décroissant (i.e. de la plus prioritaire à la moins prioritaire) :

- tâche 0 - la tâche de maintien des pieds fixes ;

- tâche 1 - la tâche de contrôle du CoM de l'exosquelette 1 ;
- tâche 2 - la tâche de contrôle du bassin de l'exosquelette 1 ;
- tâche 3 - la tâche de contrôle de la posture de l'exosquelette 1.

On pourra mettre en œuvre les combinaisons de taches suivantes : tâches 0 et 1, tâches 0 et 2, tâches 0 et 3, tâches 0, 1 et 2, tâches 0, 1 et 3, tâches 0, 2 et 3, et tâches 0, 1, 2 et 3.

On comprend que dans l'ordre l'exosquelette 1 cherche avant tout à garder les pieds au sol, puis à placer le CoM comme désiré et de manière stable, et enfin, si les deux premières conditions sont remplies, à placer au mieux le bassin comme désiré puis la posture globale comme désiré.

On verra plus loin en détail des exemples de boucles de contrôle pour chacune de ces tâches.

Procédé

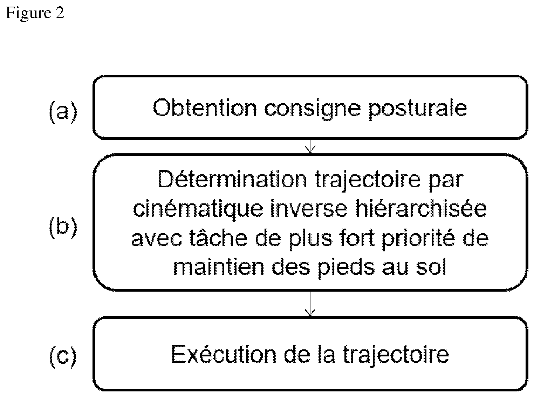

En référence à la **figure 2,** ledit procédé de mise en mouvement de l'exosquelette 1, mis en œuvre par les moyens de traitement de données 11 embarqués, commence par une étape (a) d'obtention d'au moins une consigne posturale à appliquer par l'exosquelette 1, pour que l'opérateur réalise un mouvement d'exercice. Par consigne posturale, on entend une consigne de CoM et/ou d'articulation définissant la pose désirée de l'exosquelette. Par exemple, pour un squat on a une consigne de plier les genoux. On comprend que chaque consigne posturale peut être d'intérêt pour toute ou partie des tâches. Par exemple, la consigne de CoM est bien entendu d'intérêt pour la tâche de contrôle du CoM mais pas celle de maintien des pieds au sol.

De manière connue, l'étape (a) peut comprendre l'identification d'une intention de mouvement par l'opérateur, à partir de données acquises par des capteurs ; et la détermination de la consigne posturale à partir de ladite intention de mouvement, tout en respectant des contraintes.

Pour identifier l'intention de mouvement, l'opérateur peut être muni comme expliqué d'un gilet de capteurs 15 permettant de détecter la configuration de son buste (orientation de celui-ci) et/ou une télécommande.

La consigne du centre de masse peut quant à elle être déterminée grâce aux moyens de mesure inertielle 14.

L'idée est avantageusement de calculer par exemple la consigne à partir un angle de tangage et/ou un angle de roulis du torse de l'opérateur déterminés à partir des mesures inertielles (notés *angle_tangage* et *angle_roulis*), éventuellement après comparaison avec un seuil prédéterminé, notamment pour l'angle de tangage. Si on est en dessous du seuil, l'exosquelette 1 est en mode « balance » : il accompagne les mouvements gauche/droite et avant/arrière de l'opérateur tout en maintenant la stabilité du système.

On peut appliquer une loi proportionnelle et calculer la consigne $c^t$ de CoM de la manière suivante : $c_x = K_t * angle\_tangage$ et : $c_y = K_r * angle\_roulis$.

Au-dessus de ce même seuil pour l'angle de tangage, l'exosquelette est en mode « squat » : plus l'angle est important, plus l'exosquelette se met dans une posture de semi-squat : on peut garder la loi proportionnelle pour le roulis (i.e. $c_y = K_r * angle\_roulis$) mais on utilise avantageusement pour le tangage une loi géométrique reliant l'angle de tangage à une posture menant un semi-squat, et on a alors une consigne posturale portant sur des articulations de l'exosquelette 1 définissant ladite posture de semi-squat, plutôt qu'une consigne particulière sur $c_x$.

De la même manière, pour rendre les mouvements plus anthropomorphes et agréables pour l'opérateur, l'exosquelette peut accompagner les mouvements de flexions latérales du dos et calculer une consigne $c^t$ de pose du bassin en particulier également par une loi proportionnelle : $C_{angle\_roulis\_bassin} = K_r * angle\_roulis$.

On comprendra qu'on n'est pas limité à une stratégie particulière de définition des consignes posturales, et que le nombre et la nature de ces consignes peut être très différent d'un mouvement d'exercice à un autre. L'homme du métier saura définir les consignes posturales de son choix, et l'exosquelette 1 saura dans tous les cas les utiliser.

Ensuite, dans une étape (b), comme expliqué on détermine une trajectoire de l'exosquelette 1 lors de laquelle les pieds restent au sol par la mise en œuvre de ladite cinématique inverse hiérarchisée en fonction de la ou les consignes posturales déterminées, avec la tâche de plus forte priorité qui est la tâche de maintien des pieds de l'exosquelette 1 au sol lors dudit mouvement d'exercice de l'opérateur.

De manière préférée, l'étape (b) peut en outre comprendre préalablement la conversion de la consigne de centre de masse en une consigne d'accélération du centre de masse via un algorithme de stabilisation et compensation des « flexibilités ».

Plus précisément, l'exosquelette 1 ne peut pas être considéré comme un « robot rigide », c'est-à-dire un système articulé dont la dynamique peut être suffisamment bien décrite par les équations classiques de la robotique rigide:

- L'opérateur dans l'exosquelette 1 est lui-même une source de perturbations, potentiellement importantes ;
- Des parties de l'exosquelette sont déformables (en particulier cheville et/ou hanche), ce qui se traduit notamment par le fait que lorsque l'opérateur se penche sur le côté, le CoM est généralement plus loin et peut potentiellement sortir de sa zone de stabilité.

Le présent procédé résout très astucieusement ces difficultés en prenant en compte un modèle de flexibilité de l'exosquelette 1 par rapport à un robot rigide pour ladite conversion de consigne.

A ce titre, ladite tâche de contrôle du CoM de l'exosquelette 1 est préférentiellement une tâche de contrôle en position et de stabilisation du CoM via un contrôleur basé sur un modèle de pendule inversé flexible, voir par exemple le document Estimation and Stabilization of Humanoid Flexibility Deformation Using Only Inertial Measurement Units and Contact Information. Mehdi Benallegue, Florent Lamiraux. International Journal of Humanoid Robotics, World Scientific Publishing, 2015.

Enfin, dans une étape (c), ladite trajectoire est exécutée pour accomplir le mouvement d'exercice.

Boucles de contrôle des tâches

De manière connue, le procédé comprend l'exécution de boucles de contrôle définissant pour chaque tâche l'évolution de la position de l'exosquelette 1 (i.e. le vecteur des positions articulaires) de sorte à mettre en œuvre ladite trajectoire déterminée, i.e. de sorte à ce que l'exosquelette 1 bouge. On comprend que l'exécution de ces boucles permet la mise en œuvre en temps réel des étapes (b) et (c).

Globalement, on a une boucle par tâche, et la cinématique inverse hiérarchisée est en pratique mise en œuvre au sein desdites boucles de contrôle.

Ainsi, on a par exemple pour la tâche 0 une boucle de contrôle sur la position/orientation des pieds, pour la tâche 1 une boucle de contrôle sur la position du CoM, pour la tâche 2 une boucle de contrôle sur l'orientation du bassin et/ou pour la tâche 3 une boucle de contrôle sur la posture.

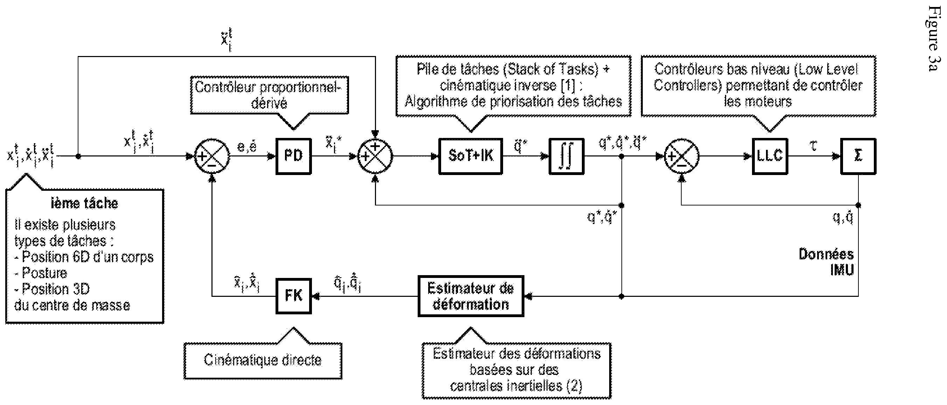

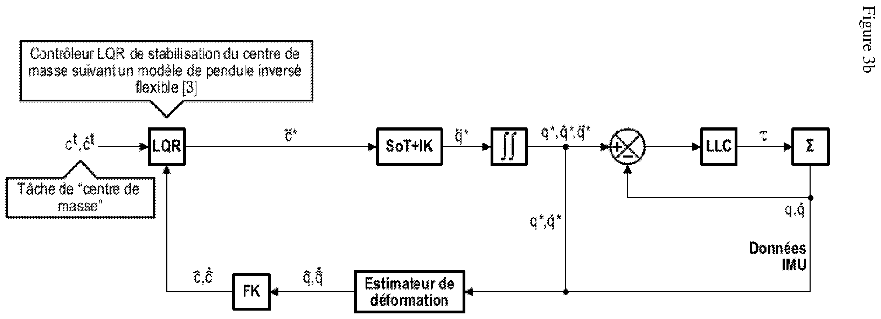

En référence aux **figures 3a et 3b** on peut avoir deux types de boucles de contrôle :

- La première boucle de contrôle, dont un exemple est illustré par la figure 3a, est pour la tâche de maintien des pieds de l'exosquelette 1 au sol, ainsi que les éventuelles tâches de contrôle du bassin de l'exosquelette et de contrôle de la posture de l'exosquelette 1 ;
- La deuxième boucle de contrôle, dont un exemple est illustré par la figure 3b, est spécifique à la tâche de contrôle du CoM.

On va à présent décrire plus en détail un mode de réalisation de chacune des boucles, même si on comprendra que toute boucle de contrôle prenant en entrée, pour une tâche donnée, la ou les consignes posturales d'intérêt vis-à-vis de la tâche, et mettant un œuvre la cinématique inverse et au moins un contrôleur pourra être utilisée.

Dans l'exemple de la figure 3a (première boucle), on note $x_i^t$, $\dot{x}_i^t$, $\ddot{x}_i^t$ les consignes posturales d'intérêt vis-à-vis de la tâche, en particulier des positions/orientations attendues d'un corps ou d'une partie de l'exosquelette 1 impliqué dans la tâche, leurs dérivées (vitesses) et dérivées secondes (accélérations). Par exemple, pour la tâche de maintien des pieds de l'exosquelette 1 au sol, il s'agit de la position/orientation des pieds (6D) qui doit rester constante (i.e. dérivées et dérivées secondes nulles).

Ensuite la boucle comprend le calcul une erreur notée e, ė entre ces consignes et les valeurs estimées correspondantes dans l'état courant de l'exosquelette 1

$$\widehat{x}_i^{\;t}, \dot{\widehat{x}}_i^{\;t}$$ (i.e. les positions/orientations estimées du corps ou de la partie de l'exosquelette 1 impliqué dans la tâche, par exemple la position estimée des pieds de l'exosquelette 1).

Un premier contrôleur, par exemple Proportionnel Dérivé (PD) calcule une commande $\ddot{x}_i^*$ à partir des erreurs et de positions/vitesses à appliquer (dites cibles) des degrés actionnés $q^*,\dot{q}^*$.

La cinématique inverse hiérarchisée peut alors être mise en œuvre sur la pile de tâches (IK + SoT), préférentiellement en appliquant l'algorithme proposé dans le document mentionné ci-avant A Dedicated Quadradic Program for Fast Hierarchical-Inverse-Kinematic Resolution. A. Escande, N. Mansard and P-B. Wieber. In IEEE Int. Conf. on Robotics and Automation (ICRA'10), Anchorage, USA, May 2010, pour justement déterminer des accélérations cibles des degrés actionnés $\ddot{q}^*$, et une ou deux intégrations permettent de retrouver les positions/vitesses cibles des degrés actionnés $q^*,\dot{q}^*$.

Un contrôleur bas niveau (LLC, Low Level Controller) permet de contrôler les actionneurs de l'exosquelette sur la base des cibles $q^*$, $\dot{q}^*$, $\ddot{q}^*$ et de l'état instantané $q$, $\dot{q}$ des degrés de liberté de l'exosquelette 1.

De manière avantageuse, un estimateur de déformation basé sur des mesures inertielles estime des positions/vitesses « réelles » des degrés actionnés

$$\hat{q}, \; \dot{\hat{q}}$$ (correspondant à une correction des valeurs $q$, $\dot{q}$ en

prenant en compte les flexibilités), en utilisant par exemple l'algorithme décrit dans le document Vigne, Matthieu, et al. "State Estimation for a Legged Robot With Multiple Flexibilities Using IMU s: A Kinematic Approach." IEEE Robotics and Automation Letters 5.1 (2019): 195-202.

Enfin, une cinématique directe permet d'en déduire les valeurs estimées $$\widehat{x}_i^{\;t}, \dot{\widehat{x}}_i^{\;t}$$ correspondant aux

consignes (i.e. les positions/orientations du corps ou de la partie de l'exosquelette 1 impliqué dans la tâche).

Dans l'exemple de la figure 3b, on note $c^t$, $\dot{c}^t$ les consignes posturales d'intérêt vis-à-vis de la tâche de contrôle du CoM, i.e. la consigne de CoM. On note qu'on n'a pas encore de consigne d'accélération puisque dans le mode préféré on la dérive via un algorithme de stabilisation et compensation des flexibilités de l'exosquelette 1, qui est avantageusement ledit contrôleur basé sur un modèle de pendule inversé flexible, avantageusement de type linéaire-quadratique (LQR, Linear-quadratic regulator). La sortie $\ddot{c}^*$ une commande au même titre que $\ddot{x}_i^*$ pour les autres tâches.

De la même manière que dans la première boucle, la cinématique inverse hiérarchisée peut alors être mise en œuvre sur la pile de tâches (IK + SoT), préférentiellement en appliquant l'algorithme proposé dans le document mentionné ci-avant A Dedicated Quadradic Program for Fast Hierarchical-Inverse-Kinematic Resolution. A. Escande, N. Mansard and P-B. Wieber. In IEEE Int. Conf. on Robotics and Automation (ICRA'10), Anchorage, USA, May 2010, pour justement déterminer des accélérations cibles des degrés actionnés $\ddot{q}^*$, et une ou deux intégrations permettent de retrouver les positions/vitesses cibles des degrés actionnés $q^*, \dot{q}^*$.

Le même contrôleur bas niveau (LLC, Low Level Controller) permet de contrôler les actionneurs de l'exosquelette sur la base des cibles $q^*$, $\dot{q}^*$, $\ddot{q}^*$ et de l'état instantané $q$, $\dot{q}$ des degrés de liberté de l'exosquelette 1.

De manière avantageuse, un estimateur de déformation basé sur des mesures inertielles estime à nouveau des positions/vitesses « réelles » des degrés

actionnés $\hat{q}$, $\dot{\hat{q}}$ (correspondant à une correction des

valeurs $q$, $\dot{q}$ en prenant en compte les flexibilités), en utilisant par exemple l'algorithme décrit dans le document Vigne, Matthieu, et al. "State Estimation for a Legged Robot With Multiple Flexibilities Using IMU s: A Kinematic Approach." IEEE Robotics and Automation Letters 5.1 (2019): 195-202.

Enfin, une cinématique directe permet d'en déduire les valeurs estimées $\hat{c}^t$, $\dot{\hat{c}}^t$ correspondant aux consignes (i.e. la position/vitesse estimée du CoM).

Equipements et système

Selon un deuxième aspect, l'invention concerne l'exosquelette 1, pour la mise en œuvre du procédé selon le premier aspect.

L'exosquelette 1 comprend des moyens de traitement de données 11 configurés pour la mise en œuvre du procédé selon le premier aspect, ainsi que si nécessaire des moyens de stockage de données 12, des moyens de mesure inertielle 14 (centrale à inertie), des moyens pour détecter l'impact des pieds au sol 13 (capteurs de contact ou éventuellement capteurs de pression), et/ou un gilet muni de capteurs 15.

Il présente une pluralité de degrés de liberté dont au moins un degré de liberté actionné par un actionneur commandé par les moyens de traitement de données 11.

Produit programme d'ordinateur

Selon un troisième et un quatrième aspects, l'invention concerne un produit programme d'ordinateur comprenant des instructions de code pour l'exécution (sur les moyens de traitement 11), d'un procédé selon le premier aspect de de mise en mouvement d'un exosquelette 1, ainsi que des moyens de stockage lisibles par un équipement informatique sur lequel on trouve ce produit programme d'ordinateur.

## Revendications

1. Procédé de mise en mouvement d'un exosquelette (1) bipède recevant un opérateur humain, le procédé comprenant la mise en œuvre par des moyens de traitement de données (11) de l'exosquelette (1), d'étapes de :

   (a) obtention d'au moins une consigne posturale à appliquer par l'exosquelette (1) pour que l'opérateur réalise un mouvement d'exercice ;
   (b) détermination d'une trajectoire de l'exosquelette (1) ;

   **caractérisé en ce que** la détermination d'une trajectoire de l'exosquelette (1) s'effectue par la mise en œuvre d'une cinématique inverse hiérarchisée en fonction de l'au moins une consigne posturale déterminée, ladite cinématique inverse hiérarchisée présentant une pile de tâches hiérarchisées comprenant, comme tâche de plus forte priorité, une tâche de maintien des pieds de l'exosquelette (1) au sol lors dudit mouvement d'exercice de l'opérateur.

2. Procédé selon la revendication 1, dans lequel la pile de tâches hiérarchisées comprend en outre au moins une tâche de contrôle du reste de l'exosquelette (1), de priorité moindre que ladite tâche de maintien des pieds de l'exosquelette (1) au sol.

3. Procédé selon la revendication 2, dans lequel ladite au moins une tâche de contrôle du reste de l'exosquelette (1) est choisie parmi

   - une tâche de contrôle du centre de masse, CoM, de l'exosquelette (1) ;
   - une tâche de contrôle du bassin de l'exosquelette (1) ;
   - une tâche de contrôle de la posture de l'exosquelette (1).

4. Procédé selon la revendication 3, dans laquelle la pile de tâches hiérarchisées comprend, par ordre de priorité décroissant, ladite tâche de maintien des pieds de l'exosquelette (1) au sol, la tâche de contrôle du CoM de l'exosquelette (1), la tâche de contrôle du bassin de l'exosquelette (1), et la tâche de contrôle de la posture de l'exosquelette (1).

5. Procédé selon l'une des revendications 3 et 4, dans lequel la tâche de contrôle du CoM de l'exosquelette (1) est une tâche de contrôle en position et de stabilisation du CoM via un contrôleur basé sur un modèle de pendule inversé flexible.

6. Procédé selon l'une des revendications 1 à 5, dans lequel l'étape (b) comprend l'exécution de boucles de contrôle définissant pour chaque tâche l'évolution d'une position de l'exosquelette (1) de sorte à mettre en œuvre ladite trajectoire déterminée.

7. Procédé selon la revendication 6, dans lequel on a une boucle de contrôle indépendante par tâche, la cinématique inverse hiérarchisée étant mise en œuvre au sein desdites boucles de contrôle.

8. Procédé selon l'une des revendications 6 à 7, dans lequel la position de l'exosquelette (1) est définie par un vecteur des positions articulaires des degrés de liberté actionnés de l'exosquelette (1).

9. Procédé selon l'une des revendications 1 à 8, dans lequel l'étape (a) comprend l'identification d'une intention de mouvement par l'opérateur, à partir de données acquises par des capteurs de l'exosquelette (1) ; et la détermination de l'au moins une consigne posturale à partir de ladite intention de mouvement.

10. Procédé selon l'une des revendications 1 à 9, dans lequel l'au moins une consigne posturale est une consigne de centre de masse, CoM, et/ou d'articulation définissant une pose désirée de l'exosquelette (1) lors dudit mouvement d'exercice de l'opérateur.

11. Exosquelette (1) comprenant des moyens de traitement de données (11) configurés pour mettre en œuvre un procédé selon l'une des revendications 1 à 10 de mise en mouvement de l'exosquelette (1).

12. Produit programme d'ordinateur comprenant des instructions de code pour l'exécution d'un procédé selon l'une des revendications 1 à 10 de mise en mouvement d'un exosquelette (1), lorsque ledit programme est exécuté sur un ordinateur.

13. Moyen de stockage lisible par un équipement informatique sur lequel un produit programme d'ordinateur comprend des instructions de code pour l'exécution d'un procédé selon l'une des revendications 1 à 10 de mise en mouvement d'un exosquelette (1).

## Patentansprüche

1. Verfahren zum Bewegen eines zweibeinigen Exoskeletts (1), das einen menschlichen Bediener aufnimmt, wobei das Verfahren die Umsetzung folgender Schritte durch Datenverarbeitungsmittel (11) des Exoskeletts (1) umfasst:

(a) Erhalten mindestens einer vom Exoskelett (1) anzuwendenden Haltungsvorgabe, damit der Bediener eine Übungsbewegung ausführen kann;

(b) Bestimmen einer Bahn des Exoskeletts (1);

**dadurch gekennzeichnet, dass** die Bestimmung einer Bahn des Exoskeletts durch die Umsetzung einer hierarchischen inversen Kinematik in Abhängigkeit von der mindestens einen bestimmten Haltungsvorgabe erfolgt, wobei die hierarchische inverse Kinematik einen Stapel hierarchischer Aufgaben aufweist, der als Aufgabe mit der höchsten Priorität eine Aufgabe des Haltens der Füße des Exoskeletts (1) auf dem Boden während der Übungsbewegung des Bedieners umfasst.

2. Verfahren nach Anspruch 1, wobei der Stapel hierarchischer Aufgaben ferner mindestens eine Aufgabe der Steuerung des restlichen Exoskeletts (1) umfasst, die eine geringere Priorität als die Aufgabe des Haltens der Füße des Exoskeletts (1) auf dem Boden hat.

3. Verfahren nach Anspruch 2, wobei die mindestens eine Aufgabe der Steuerung des restlichen Exoskeletts (1) ausgewählt ist aus

   - einer Aufgabe der Steuerung des Schwerpunkts, CoM, des Exoskeletts (1);
   - einer Aufgabe der Steuerung des Beckens des Exoskeletts (1);
   - einer Aufgabe der Steuerung der Haltung des Exoskeletts (1).

4. Verfahren nach Anspruch 3, wobei der Stapel hierarchischer Aufgaben in absteigender Reihenfolge ihrer Priorität die Aufgabe des Haltens der Füße des Exoskeletts (1) auf dem Boden, die Aufgabe der Steuerung des CoM des Exoskeletts (1), die Aufgabe der Steuerung des Beckens des Exoskeletts (1), und die Aufgabe der Steuerung der Haltung zu steuern, umfasst.

5. Verfahren nach einem der Ansprüche 3 und 4, wobei die Aufgabe der Steuerung des CoM des Exoskeletts (1), eine Aufgabe der Positionssteuerung und der Stabilisierung des CoM über einen auf einem flexiblen umgekehrten Pendelmodell basierenden Controller ist.

6. Verfahren nach einem der Ansprüche 1 bis 5, wobei der Schritt (b) die Ausführung von Regelkreisen umfasst, die für jede Aufgabe die Entwicklung einer Position des Exoskeletts (1) so definieren, dass die festgelegte Bahn umgesetzt wird.

7. Verfahren nach Anspruch 6, wobei je Aufgabe ein unabhängiger Regelkreis vorhanden ist, wobei die hierarchische inverse Kinematik innerhalb dieser Regelkreise umgesetzt wird.

8. Verfahren nach einem der Ansprüche 6 bis 7, wobei die Position des Exoskeletts (1) durch einen Vektor der Gelenkpositionen der betätigten Freiheitsgrade des Exoskeletts (1) definiert ist.

9. Verfahren nach einem der Ansprüche 1 bis 8, wobei der Schritt (a) die Identifizierung einer Bewegungsabsicht durch den Bediener anhand von Daten umfasst, die von Sensoren des Exoskeletts (1) erfasst werden; und die Bestimmung der mindestens einen Haltungsvorgabe anhand der Bewegungsabsicht.

10. Verfahren nach einem der Ansprüche 1 bis 9, wobei die mindestens eine Haltungsvorgabe eine Vorgabe für den Schwerpunkt, CoM, und/oder für Gelenke ist, die eine gewünschte Haltung des Exoskeletts (1) während der Bewegungsübung des Bedieners definiert.

11. Exoskelett (1), das Datenverarbeitungsmittel (11) umfasst, die zur Umsetzung eines Verfahrens nach einem der Ansprüche 1 bis 10 zum Bewegen des Exoskeletts (1) ausgelegt sind.

12. Rechnerprogrammprodukt, das Codebefehle zur Ausführung eines Verfahrens nach einem der Ansprüche 1 bis 10 zum Bewegen eines Exoskeletts (1) umfasst, wenn das Programm auf einem Rechner ausgeführt wird.

13. Speichermedium, das von einer IT-Ausrüstung lesbar ist, auf dem ein Rechnerprogrammprodukt Codebefehle zur Ausführung eines Verfahrens nach einem der Ansprüche 1 bis 10 zum Bewegen eines Exoskeletts (1) umfasst.

**Claims**

1. A method for moving a bipedal exoskeleton (1) accommodating a human operator, the method including the implementation, by data processing means (11) of the exoskeleton (1), of steps of:

   (a) obtaining at least one postural instruction to be applied by the exoskeleton (1) in order for the operator to perform an exercise movement;
   (b) determining a trajectory of the exoskeleton (1)

   **characterized in that** determining a trajectory of the exoskeleton (1) is done by means of hierarchized inverse kinematics based on the at least one determined postural instruction, said hierarchized inverse kinematics comprising a stack of hierarchized tasks comprising, as the task of highest priority, a task of keeping the feet of the exoskeleton (1) on the ground during said exercise movement of the operator.

2. The method according to claim 1, wherein the stack of hierarchized tasks further comprises at least one task of controlling the rest of the exoskeleton (1), of lower priority than said task of keeping the feet of the exoskeleton (1) on the ground.

3. The method according to claim 2, wherein said at least one task of controlling the rest of the exoskeleton (1) is selected from

    - a task of controlling the center of mass, CoM, of the exoskeleton (1);
    - a task of controlling the pelvis of the exoskeleton (1);
    - a task of controlling the posture of the exoskeleton (1).

4. The method according to claim 3, wherein the stack of hierarchized tasks comprises, in decreasing order of priority, said task of keeping the feet of the exoskeleton (1) on the ground, the task of controlling the CoM of the exoskeleton (1), the task of controlling the pelvis of the exoskeleton (1), and the task of controlling the posture of the exoskeleton (1).

5. The method according to one of claims 3 and 4, wherein the task of controlling the CoM of the exoskeleton (1) is a task of CoM position control and stabilization via a controller based on a flexible inverted pendulum model.

6. The method according to one of claims 1 to 5, wherein step (b) comprises executing control loops defining for each task the evolution of a position of the exoskeleton (1) so as to implement said determined trajectory.

7. The method according to claim 6, wherein there is an independent control loop per task, the hierarchized inverse kinematics being implemented within said control loops.

8. The method according to one of claims 6 and 7, wherein the position of the exoskeleton (1) is defined by a vector of the joint positions of the actuated degrees of freedom of the exoskeleton (1).

9. The method according to one of claims 1 to 8, wherein step (a) comprises identifying a movement intention by the operator, from data acquired by sensors of the exoskeleton (1); and determining the at least one postural instruction from said movement intention.

10. The method according to one of claims 1 to 9, wherein the at least one postural instruction is a center of mass, CoM, and/or joint instruction defining a desired pose of the exoskeleton (1) during said exercise movement of the operator.

11. An exoskeleton (1) comprising data processing means (11) configured to implement a method according to one of claims 1 to 10 for moving the exoskeleton (1).

12. A computer program product comprising code instructions for executing a method according to one of claims 1 to 10 for moving an exoskeleton (1), when said program is executed on a computer.

13. A storage means readable by a computer equipment on which a computer program product comprises code instructions for executing a method according to one of claims 1 to 10 for moving an exoskeleton (1).

Figure 1

Figure 2

(a) Obtention consigne posturale

(b) Détermination trajectoire par cinématique inverse hiérarchisée avec tâche de plus fort priorité de maintien des pieds au sol

(c) Exécution de la trajectoire

Figure 3a

Figure 3b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015140352 A

- WO 2015140353 A

**Littérature non-brevet citée dans la description**

- **L. PENCO** ; **E. M. HOFFMAN** ; **V. MODUGNO** ; **W. GOMES** ; **J. -B. MOURET** ; **S. IVALDI**. Learning Robust Task Priorities and Gains for Control of Redundant Robots. *IEEE Robotics and Automation Letters*, April 2020, vol. 5 (2), 2626-2633

- **A. ESCANDE** ; **N. MANSARD** ; **P-B. WIEBER**. A Dedicated Quadradic Program for Fast Hierarchical-Inverse-Kinematic Resolution. *IEEE Int. Conf. on Robotics and Automation (ICRA'10), Anchorage, USA*, May 2010

- Estimation and Stabilization of Humanoid Flexibility Deformation Using Only Inertial Measurement Units and Contact Information. **MEHDI BENALLEGUE** ; **FLORENT LAMIRAUX**. International Journal of Humanoid Robotics. World Scientific Publishing, 2015

- **VIGNE, MATTHIEU et al.** State Estimation for a Legged Robot With Multiple Flexibilities Using IMU s: A Kinematic Approach. *IEEE Robotics and Automation Letters*, 2019, vol. 5 (1), 195-202